# EUROPEAN PATENT SPECIFICATION

(11) **EP 2 397 558 B1**
(45) Date of publication and mention of the grant of the patent: **25.02.2015**
(21) Application number: 10741033.4
(22) Date of filing: 25.01.2010
(51) Int. Cl.: C12Q 1/00

(54) **ENZYME REACTION REAGENT, ENZYME REACTION REAGENT KIT AND METHOD FOR STORING LIQUID FOR ENZYMATIC REACTION**
ENZYMREAKTIONSREAGENS, ENZYMREAKTIONSREAGENZIENKIT UND VERFAHREN ZUR FLÜSSIGKEITSLAGERUNG FÜR ENZYMATISCHE REAKTIONEN
RÉACTIF DE RÉACTION ENZYMATIQUE, KIT DE RÉACTIFS DE RÉACTION ENZYMATIQUE ET PROCÉDÉ DE STOCKAGE DE LIQUIDE POUR RÉACTION ENZYMATIQUE

(30) Priority: 13.02.2009 JP 2009031876
(43) Date of publication of application: 21.12.2011
(73) Proprietor: Taiyo Nippon Sanso Corporation, Tokyo 142-8558 (JP)
(72) Inventor: FUKUDA, Kenji, Tokyo 142-8558 (JP); OKANO, Satoshi, Tokyo 142-8558 (JP)
(74) Representative: Lamb, Martin John Carstairs
(86) International application number: PCT/JP2010/000394
(87) International publication number: WO 2010/092753

(56) References cited:
- JP-A- 7 043 362
- JP-A- 10 327 895
- JP-A- 2009 106 221
- JP-T- 2000 509 486
- JP-T- 2002 527 751
- JP-T- 2008 501 331
- RAMSAY J R ET AL: "The use of a layering technique for enhancing stability of lyophilized reagents", ANALYTICAL BIOCHEMISTRY, ACADEMIC PRESS INC, NEW YORK, vol. 202, no. 2, 1 May 1992 (1992-05-01), pages 331-336, XP024819754, ISSN: 0003-2697, DOI: 10.1016/0003-2697(92)90113-L [retrieved on 1992-05-01]
- "EasyXpress Protein Synthesis Handbook", , 1 April 2008 (2008-04-01), XP55030903, Retrieved from the Internet: URL:www.qiagen.com [retrieved on 2012-06-25]
- "Freeze-drying", Wikipedia, the free encyclopedia, 14 February 2013 (2013-02-14), XP055053474, Retrieved from the Internet: URL:http://en.wikipedia.org/wiki/Lyophiliz ation [retrieved on 2013-02-14]
- Rowe, Terence W G and Snowman, John W: "Edwards Freeze-Drying Handbook", 1978, Edwards High Vacuum, Crawley
- Philip Babcock Gove, Ph.D., Editor-in-Chief: "Webster's Third New International Dictionary of the English language, unabridged", 1993, Merriam-Webster, Cologne ISBN: 3-8290-5292-8 page 806,

## Description

### TECHNICAL FIELD

The present invention relates to an enzymatic reaction reagent, an enzymatic reaction reagent kit containing the reagent, and a method for storing a liquid for the enzymatic reaction.

### BACKGROUND ART

Enzymes are catalysts formed mainly from proteins, and catalyze chemical reactions such as oxidation, transfer, hydrolysis and various synthesis or isomerization reactions that are required for vital activities in vivo. Because these enzymatic reactions tend to proceed under more moderate conditions of temperature and pH and the like when compared with non-enzymatic reactions, they are useful for medical diagnosis or material production or the like, and are currently in widespread use.

Enzymes that are currently available commercially are supplied in a variety of forms, including powders, aqueous solutions and glycerol solutions. In consideration of usability when performing reactions using the enzyme, many of these commercially available enzymes are supplied as an enzymatic reaction reagent kit composed of a plurality of separate reagents. For example, the reagents included within an enzymatic reaction reagent kit may include one or more enzymes, buffer solutions, reducing agents, phosphoric acid sources or inhibitors or the like, and examples of these kits include enzyme antibody kits for diagnosing bovine spongiform encephalopathy, restriction enzyme kits, reverse transcriptase enzyme kits, and protein synthesis kits.

However, in those cases where a plurality of reagents included within an enzymatic reaction reagent kit are all mixed together in advance and then stored in a single container, a variety of problems may arise, including undesirable progression of the targeted enzymatic reaction, or enzymatic reactions other than the targeted reaction, during mixing or storage, and deterioration in the enzyme activity within the targeted enzymatic reaction. These problems occur even during frozen storage at low temperatures of approximately -80°C. Accordingly, enzymatic reaction reagent kits composed of a plurality of reagents are generally supplied in a form wherein the reagents are encased in a plurality of containers, with each container holding either a single reagent or a constituent liquid containing a combination of reagents that does not suffer the types of problems described above upon mixing. For example, Japanese Unexamined Patent Application, First Publication No. Hei 10-327895 discloses a kit in which a liquid reagent is divided into two reagents, namely a first reagent and a second reagent. J. R. Ramsay et al., Analytical Biochemistry, 202, 331-336 (1992) discloses a process that involves freeze-drying of an enzyme extracted from a homogenized bacterial cell paste. Rowe, Terence, W G and Snowman, John W: "Edwards Freeze-Drying Handbook", 1978 discloses methods of freeze-drying.

### CITATION LIST

### PATENT DOCUMENTS

### [Patent Document 1]

Japanese Unexamined Patent Application, First Publication No. Hei 10-327895

### DISCLOSURE OF INVENTION

### PROBLEMS TO BE SOLVED BY THE INVENTION

Conventional enzymatic reaction reagent kits suffer from the types of problems outlined below.

Namely, these enzymatic reaction reagent kits require that various operations such as thawing, dispensing and mixing, as well as pipetting of very small amounts of liquids of several µL to several tens of µL are performed for a plurality of liquids. In other words, the operations are complex and require considerable time and effort. Accordingly, fluctuations in the enzymatic reaction can occur as a result of differences in the proficiency of the operator.

Liquids that contain an enzyme frequently have high viscosity. Consequently, during the pipetting operation mentioned above, the liquid containing the enzyme tends to stick to and remain on the inner walls of the container, and therefore the total volume of the liquid within the container cannot be used, resulting in liquid loss. Because a plurality of containers are used, the raw material costs tend to increase. Freeze drying is one known method for storing enzymes and biogenic components, but preparation of reagents using freeze drying requires considerable time and effort, and the operations required for using freeze drying are complex. Further, the number of enzymes and biogenic components capable of withstanding freeze drying is small, and most of those components suffer a significant reduction or loss in activity.

The present invention takes the above circumstances into consideration, with an object of providing an enzymatic reaction reagent that exhibits excellent storage stability of the enzyme, can simplify the operations required during use of the reagent, and can reduce reagent loss and raw material costs, as well as providing an enzymatic reaction reagent kit that includes the above reagent, and a method for storing a liquid for an enzymatic reaction.

### MEANS TO SOLVE THE PROBLEMS

As a result of intensive research aimed at achieving the above object, the inventors of the present invention discovered that by storing an enzyme and one or more components capable of reacting with the enzyme, optionally and one or more components capable of reducing the enzyme activity of the enzyme, within a single container in a state that reduces the contact between the enzyme and the other components, the targeted enzyme activity could be stably maintained, and they were thus able to complete the present invention.

In other words, the present invention provides an enzymatic reaction reagent, an enzymatic reaction reagent kit, and a method for storing a liquid for an enzymatic reaction that exhibit the features described below.
(1) An enzymatic reaction reagent as claimed in claim 1.
(2) The enzymatic reaction reagent according to (1) above, wherein a second component, which is different from the first component and is capable of reducing the activity of the enzyme, is provided within a constituent liquid that does not contain the enzyme.
(3) The enzymatic reaction reagent according to (1) above, wherein the reagent is used for protein synthesis.
(4) An enzymatic reaction reagent kit containing the enzymatic reaction reagent according to any one of (1) to (3) above.
(5) A method for storing a liquid for an enzymatic reaction as defined in claim 5.

### EFFECT OF THE INVENTION

According to the present invention, the plurality of constituent liquids are frozen individually, and therefore the enzyme can be stored in a stable manner. Further, if the constituent liquids in the container are thawed, then the enzymatic reaction can be conducted immediately, meaning operation can be simplified considerably. Moreover, as a result of this operational simplification, the enzymatic reaction can be performed stably and rapidly, regardless of the proficiency of the operator. Further, because only a single container is required, reagent loss and raw material costs can be reduced. Furthermore, because freeze drying is not required, a wide variety of enzymes and biogenic components can be stored in a stable manner, thus offering excellent versatility.

### BRIEF DESCRIPTION OF THE DRAWINGS

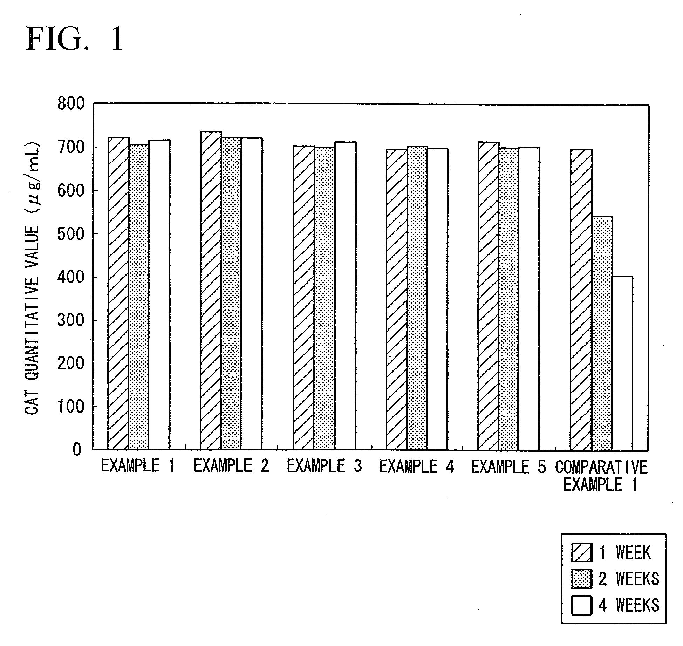

FIG. 1 is a graph illustrating quantitative values for chloramphenicol acetyltransferase (CAT) for various storage periods of enzymatic reaction reagents according to examples 1 to 5 and a comparative example 1.

### EMBODIMENTS FOR CARRYING OUT THE INVENTION

### <Enzymatic Reaction Reagent>

In the enzymatic reaction reagent of the present invention, a "constituent liquid" describes one of a plurality of separated liquid units, wherein each unit contains one or more components necessary for performing the enzymatic reaction. Each constituent liquid contains at least one of the above components, and preferably also contains a vehicle such as a solvent for the component. Although there are no particular limitations on the type of vehicle used, water is typical, but other vehicles may be included as necessary.

By mixing all of the constituent liquids, an enzyme-containing liquid for performing the target enzymatic reaction is obtained.

There are no particular limitations on the enzyme in the enzymatic reaction reagent of the present invention, and both naturally derived enzymes and artificially modified or synthesized enzymes may be used. Specifically, the enzyme may be selected appropriately from among synthase enzymes, degrading enzymes, oxidase enzymes, reductase enzymes, transferase enzymes and isomerase enzymes and the like, in accordance with the intended purpose. Examples of preferred enzymes include enzymes for synthesizing or degrading biopolymers such as DNA, RNA and proteins, or other biologically derived biomolecules besides the above biopolymers, and enzymes for synthesizing or degrading non-natural molecules other than the biopolymers and biomolecules mentioned above. Specifically, polymerases such as DNA polymerase and RNA polymerase, and aminoacyl tRNA synthase and the like are preferred, and of these, aminoacyl tRNA synthase or the like is particularly desirable.

At least one of the constituent liquids contains an enzyme, and the components contained within each of the constituent liquids can be adjusted appropriately in accordance with the type of enzymatic reaction. However, a first component capable of reacting with the enzyme is included within a constituent liquid that does not contain the enzyme. Examples of this first component include substrates for the enzyme, and other essential components for the reaction between the enzyme and the substrates. Examples of such essential components include the template nucleic acids required during synthesis of DNA or RNA, the 20 types of amino acid required when synthesizing protein, and tRNA and the like.

The reaction of the above first component with the enzyme is inhibited unless all of the constituent liquids are mixed together, and therefore by including the first component in a different constituent liquid from the enzyme, the storage stability of the enzyme can be further improved.

Moreover, a second component, which is different from the first component and is capable of reducing the activity of the enzyme, is preferably included within a constituent liquid that does not contain the enzyme. Examples of this second component include components, besides the first component, that interact with the enzyme, such as components that modify the enzyme as a result of the interaction, and components that do not modify the enzyme but form a stable complex with the enzyme. Specifically, the second component includes components which, upon performing the targeted enzymatic reaction following storage together with the enzyme for 4 weeks at a predetermined temperature, yield an enzyme activity that has been reduced by at least 40% compared with the case where the targeted enzymatic reaction is performed following separate storage of the second component and the enzyme.

The enzyme, the first component and the second component may each be either a single component or a combination of two or more components. In the case of two or more components, the combination of components and the relative proportions of each component may be selected appropriately according to the intended purpose.

Each constituent liquid contains at least one component that is necessary for performing the enzymatic reaction. Further, each constituent liquid may also contain other components, provided they do not impair the effects of the present invention. Examples of these other components include components for improving the stability of the constituent liquid, such as antioxidants and the like.

The components within the constituent liquids may be organic compounds, inorganic compounds or ions or the like. At least one of the constituent liquids includes extracts obtained from microbes or cells that contain the targeted enzyme. [0020]

There are no particular limitations on the concentration of each component contained within each of the constituent liquids, and the concentration may be altered as required.

The volume of each constituent liquid may also be adjusted arbitrarily in accordance with the intended purpose. For example, in the case of protein synthesis or the like, very small volumes of 1 mL or less may be used.

There are no particular limitations on the number of constituent liquids within the enzymatic reaction reagent provided the number is at least two, and the number may be adjusted appropriately in accordance with the type of enzymatic reaction.

The plurality of constituent liquids are frozen in a state of mutual contact or the constituent liquids are each formed as a frozen layer in a stacked configuration. The encased location of each constituent liquid can be adjusted by changing the location at which the constituent liquid is added to the container.

In the present invention, even if the frozen constituent liquids make mutual contact, the components within the constituent liquids can still be stored in a stable manner.

There are no particular limitations on the container for freezing and encasing the constituent liquids, and containers composed of conventional materials such as glasses or resins or the like can be used.

The enzymatic reaction reagent of the present invention is particularly suitable as a reagent for protein synthesis.

The enzymatic reaction reagent of the present invention can be produced by freezing each of the constituent liquids described above individually in succession, and encasing all of the constituent liquids within a single container. For example, in the case where the number of constituent liquids is n (wherein n is an integer of 2 or greater), the first constituent liquid may be added to the container and frozen, the second constituent liquid then added to the container and frozen, and this series of operations repeated until the nth constituent liquid, thereby freezing and encasing all of the constituent liquids. Further, a plurality of constituent liquids may be mixed together and used as a single constituent liquid, provided the components contained within the liquids can be store in a stable manner.

The addition of a constituent liquid to the container and subsequent freezing of the liquid must be performed in such a manner that any frozen constituent liquids already encased inside the container do not thaw. Further, the contact time between the already frozen constituent liquid inside the container and the latterly added but as yet unfrozen constituent liquid is preferably as short as possible, and subjecting the latterly added constituent liquid to snap freezing is particularly desirable. This enables the components such as the enzyme contained within the constituent liquid to be stored in an even more stable manner. In order to achieve snap freezing, the constituent liquid is preferably added to the container with the container cooled to a temperature capable of freezing the constituent liquid being added.

The temperature for freezing the constituent liquid may be adjusted appropriately in accordance with the components in the constituent liquid and the type of vehicle used. For example, the temperature may be set to -20°C or lower, and preferably -70°C or lower. Typically, a cooling medium such as liquid nitrogen or dry ice can be used. These cooling media are readily obtainable and exhibit an excellent cooling effect, and are consequently ideal.

During production of the enzymatic reaction reagent, the cooling temperature during freezing of the constituent liquids may be a constant temperature, or may be varied appropriately provided the frozen constituent liquids do not thaw. For example, in those cases where addition of a constituent liquid is performed while the container is being cooled, if the amount added is very small, then the constituent liquid may freeze at the discharge outlet of the device used for performing the addition, and therefore the temperature may be altered so that, for example, the cooling temperature is increased during addition of the constituent liquid.

By freezing each of the constituent liquids and encasing all of the constituent liquids within a container, the enzymatic reaction reagent of the present invention is obtained. By subsequently placing the enzymatic reaction reagent in frozen storage at a temperature that ensures none of the constituent liquids undergoes thawing, the enzyme and each of the other components within the constituent liquids can be stored in a stable manner. Then, when the enzymatic reaction reagent is to be used, the components necessary for the enzymatic reaction can be mixed together by simply thawing the constituent liquids, thereby yielding an enzyme-containing liquid. Other components required for the enzymatic reaction may also be added to the thus obtained enzyme-containing liquid.

The enzymatic reaction reagent of the present invention is preferably capable of completing the targeted enzymatic reaction using only the constituent liquids contained therein, but may also be applied to reactions that cannot be completed using only the enzymatic reaction reagent. For example, in the case of a cell-free protein synthesis reagent that employs the present invention, the template nucleic acid that codes the target enzymatic reaction product may be included within the reagent, but for reasons such as imparting greater versatility to the reagent, the template nucleic acid may be excluded from the reagent, and added separately. This type of substance that is not included within the enzymatic reaction reagent, but is rather added at the time of the enzymatic reaction, may be included within the enzymatic reaction reagent kit according to the present invention.

The frozen storage temperature for the enzymatic reaction reagent may be adjusted appropriately, in accordance with the components in the constituent liquid and the type of vehicle used, to a temperature that ensures none of the constituent liquids undergoes thawing. For example, the storage temperature is typically -12°C or lower, is preferably -18°C or lower, and is more preferably a temperature that is lower than the lowest recommended storage temperature for the various components and vehicles contained within the reagent. A temperature of approximately -90 to -70°C is usually adequate.

### <Enzymatic Reaction Reagent Kit>

In the present invention, the enzymatic reaction reagent kit contains the enzymatic reaction reagent described above. Further, the enzymatic reaction reagent kit may also include other arbitrary reagents other than the above enzymatic reaction reagent.

### EXAMPLES

The present invention is described below in further detail based on a series of examples, although the present invention is in no way limited by the following examples.

Differences in the yield of an enzymatic reaction product were investigated for different methods of producing the enzymatic reaction reagent.

Specifically, synthesis of chloramphenicol acetyltransferase (CAT), which can be easily quantified using a common method, was conducted using a cell-free protein synthesis method.

The components of each of the constituent liquids used in the cell-free protein synthesis method are shown in Table 1. Each of the constituent liquids is a liquid containing water as the main solvent.

Examples of first components for the T7RNA polymerase include ATP, GTP, CTP and UTP, an example of a first component for the Escherichia coli extract is the 20 amino acids mixture, and examples of first components for the creatine kinase aqueous solution include creatine phosphate and ATP.

**[Table 1]**

| Constituent liquid | Components | | |
|---|---|---|---|
| (1) | T7RNA polymerase | | |
| (2) | Escherichia coli extract | | |
| (3) | Creatine kinase aqueous solution | | |
| (4) | 20 amino acids mixture aqueous solution | | Dithiothreitol |
| (5) | Magnesium acetate aqueous solution | | |
| (6) | HEPES | D-glutamic acid | ATP, GTP, CTP, UTP |
| | Folinic acid | cAMP | Dithiothreitol |
| | Ammonium acetate | Creatine phosphate | tRNA |

### [Example 1]

13.2 µL of the constituent liquid (1) was added to a 1.5 mL polypropylene container using a pipettor, and following sealing of the container with a lid, the added constituent liquid was frozen by bringing the container into contact with liquid nitrogen. Following removal of the container from the liquid nitrogen, the container was placed on a dry ice bath, the lid was opened, 238 µL of the constituent liquid (2) was added, and the lid was then immediately closed and the added constituent liquid was frozen by bringing the container into contact with liquid nitrogen. Following removal of the container from the liquid nitrogen again, the container was placed on a dry ice bath, the lid was opened, 66 µL of the constituent liquid (3) was added, and the lid was then immediately closed and the added constituent liquid was frozen by bringing the container into contact with liquid nitrogen. In a similar manner, 74 µL of the constituent liquid (4), 79 µL of the constituent liquid (5) and 451 µL of the constituent liquid (6) were added individually to the container in succession, with each added constituent liquid being frozen by bringing the container into contact with liquid nitrogen, thereby completing preparation of an enzymatic reaction reagent. The reagent was then removed from the liquid nitrogen and stored in a freezer at -80°C for a predetermined period.

Subsequently, the container was removed from the freezer and the constituent liquids were thawed and mixed over ice, thus forming a protein synthase solution.

79 µL of pUC-CAT was added to the protein synthase solution as a template DNA, and following stirring, the mixture was heated at 37°C for one hour to synthesize CAT.

### [Example 2]

13.2 µL of the constituent liquid (1) and 238 µL of the constituent liquid (2) were added to a 1.5 mL polypropylene container using a pipettor, and following sealing of the container with a lid, the added constituent liquids were frozen by bringing the container into contact with liquid nitrogen. Following removal of the container from the liquid nitrogen, the container was placed on a dry ice bath, the lid was opened, 66 µL of the constituent liquid (3) was added, and the lid was then immediately closed and the added constituent liquid was frozen by bringing the container into contact with liquid nitrogen. In a similar manner, 74 µL of the constituent liquid (4), 79 µL of the constituent liquid (5) and 451 µL of the constituent liquid (6) were added individually to the container in succession, with each added constituent liquid being frozen by bringing the container into contact with liquid nitrogen, thereby completing preparation of an enzymatic reaction reagent. The reagent was then removed from the liquid nitrogen and stored in a freezer at -80°C for a predetermined period.

Subsequently, the container was removed from the freezer and the constituent liquids were thawed and mixed over ice, thus forming a protein synthase solution.

79 µL of pUC-CAT was added to the protein synthase solution as a template DNA, and following stirring, the mixture was heated at 37°C for one hour to synthesize CAT.

### [Example 3]

13.2 µL of the constituent liquid (1), 238 µL of the constituent liquid (2) and 66 µL of the constituent liquid (3) were added to a 1.5 mL polypropylene container using a pipettor, and following sealing of the container with a lid, the added constituent liquids were frozen by bringing the container into contact with liquid nitrogen. Following removal of the container from the liquid nitrogen, the container was placed on a dry ice bath, the lid was opened, 74 µL of the constituent liquid (4) was added, and the lid was then immediately closed and the added constituent liquid was frozen by bringing the container into contact with liquid nitrogen. In a similar manner, 79 µL of the constituent liquid (5) and 451 µL of the constituent liquid (6) were added individually to the container in succession, with each added constituent liquid being frozen by bringing the container into contact with liquid nitrogen, thereby completing preparation of an enzymatic reaction reagent. The reagent was then removed from the liquid nitrogen and stored in a freezer at -80°C for a predetermined period.

Subsequently, the container was removed from the freezer and the constituent liquids were thawed and mixed over ice, thus forming a protein synthase solution.

79 µL of pUC-CAT was added to the protein synthase solution as a template DNA, and following stirring, the mixture was heated at 37°C for one hour to synthesize CAT.

### [Example 4]

13.2 µL of the constituent liquid (1), 238 µL of the constituent liquid (2) and 66 µL of the constituent liquid (3) were added to a 1.5 mL polypropylene container using a pipettor, and following sealing of the container with a lid, the added constituent liquids were frozen by bringing the container into contact with liquid nitrogen. Following removal of the container from the liquid nitrogen, the container was placed on a dry ice bath, the lid was opened, 74 µL of the constituent liquid (4) and 79 µL of the constituent liquid (5) were added, and the lid was then immediately closed and the added constituent liquids were frozen by bringing the container into contact with liquid nitrogen. In a similar manner, 451 µL of the constituent liquid (6) was added to the container, and the added constituent liquid was frozen by bringing the container into contact with liquid nitrogen, thereby completing preparation of an enzymatic reaction reagent. The reagent was then removed from the liquid nitrogen and stored in a freezer at -80°C for a predetermined period.

Subsequently, the container was removed from the freezer and the constituent liquids were thawed and mixed over ice, thus forming a protein synthase solution.

79 µL of pUC-CAT was added to the protein synthase solution as a template DNA, and following stirring, the mixture was heated at 37°C for one hour to synthesize CAT.

### [Example 5]

13.2 µL of the constituent liquid (1), 238 µL of the constituent liquid (2) and 66 µL of the constituent liquid (3) were added to a 1.5 mL polypropylene container using a pipettor, and following sealing of the container with a lid, the added constituent liquids were frozen by bringing the container into contact with liquid nitrogen. Following removal of the container from the liquid nitrogen, the container was placed on a dry ice bath, the lid was opened, 74 µL of the constituent liquid (4), 79 µl of the constituent liquid (5) and 451 µL of the constituent liquid (6) were added, and the lid was then immediately closed and the added constituent liquids were frozen by bringing the container into contact with liquid nitrogen, thereby completing preparation of an enzymatic reaction reagent. The reagent was then removed from the liquid nitrogen and stored in a freezer at -80°C for a predetermined period.

Subsequently, the container was removed from the freezer and the constituent liquids were thawed and mixed over ice, thus forming a protein synthase solution.

79 µL of pUC-CAT was added to the protein synthase solution as a template DNA, and following stirring, the mixture was heated at 37°C for one hour to synthesize CAT.

### [Comparative Example 1]

13.2 µL of the constituent liquid (1), 238 µL of the constituent liquid (2), 66 µL of the constituent liquid (3), 74 µL of the constituent liquid (4), 79 µL of the constituent liquid (5) and 451 µL of the constituent liquid (6) were added to a 1.5 mL polypropylene container using a pipettor, thereby forming a protein synthase solution. Following sealing of the container with a lid, the added constituent liquids were frozen by bringing the container into contact with liquid nitrogen, thus completing preparation of an enzymatic reaction reagent. The reagent was then removed from the liquid nitrogen and stored in a freezer at -80°C for a predetermined period.

79 µL of pUC-CAT was added to the above-mentioned protein synthase solution as a template DNA, and following stirring, the mixture was heated at 37°C for one hour to synthesize CAT.

### [Comparative Example 2]

0.4 µL of the constituent liquid (1), 7.2 µL of the constituent liquid (2), 2 µL of the constituent liquid (3), 2.3 µL of the constituent liquid (4), 2.4 µL of the constituent liquid (5) and 13.6 µL of the constituent liquid (6) were added to a 0.6 mL polypropylene container using a pipettor, thereby forming a protein synthase solution. Following sealing of the container with a lid, the container was not frozen, but rather the protein synthase solution was immediately used for performing the enzymatic reaction.

2.4 µL of pUC-CAT was added to the protein synthase solution as a template DNA, and following stirring, the mixture was heated at 37°C for one hour to synthesize CAT.

Table 2 illustrates whether or not each of the constituent liquids was added at the same time as another constituent liquid in the above Examples 1 to 5 and Comparative Examples 1 and 2. In Table 2, those constituent liquids that are separated into different table cells by a horizontal line were not added at the same time. The constituent liquids (1) to (6) in Table 2 are the same as the constituent liquids of Table 1.

**[Table 2]**

| Constituent liquid | Example | | | | | Comparative example | |
|---|---|---|---|---|---|---|---|
| | 1 | 2 | 3 | 4 | 5 | 1 | 2 |
| (1) | | | | | | | |
| (2) | | | | | | | |
| (3) | | | | | | | |
| (4) | | | | | | | |
| (5) | | | | | | | |
| (6) | | | | | | | |
| * Comparative Example 2 was not frozen | | | | | | | |

Quantitative determination of the CAT was performed using the method described below.

3 µL of one of the reaction solutions prepared in the above Examples 1 to 5 and Comparative Examples 1 to 2 was added to a mixed liquid containing 8 µL of acetyl CoA, 352 µL of chloramphenicol and 40 of DTNB (5,5'-thiobis-2-nitrobenzoic acid), and following heating at 37°C for 30 minutes, the absorbance was measured using an ultraviolet absorption spectrophotometer (412 nm), and the quantity of CAT was determined using a conversion formula.

Table 3 and FIG. 1 illustrate the quantitative values (µg/mL) for CAT for various storage periods of the enzymatic reaction reagents according to Examples 1 to 5 and Comparative Examples 1 and 2. Comparative Example 2, in which the enzymatic reaction was performed immediately without storing the enzymatic reaction reagent, is omitted from FIG. 1.

**[Table 3]**

| Storage period | Example 1 | Example 2 | Example 3 | Example 4 | Example 5 | Comparative Example 1 | Comparative Example 2 |
|---|---|---|---|---|---|---|---|
| None | - | - | - | - | - | - | 680±25 |
| 1 week | 720 | 735 | 703 | 696 | 714 | 698 | - |
| 2 weeks | 705 | 722 | 699 | 702 | 700 | 543 | - |
| 4 weeks | 715 | 721 | 711 | 699 | 703 | 405 | - |
| 3 months | - | - | - | - | 747 | - | - |

From these results it was evident that, for all storage periods from 1 to 4 weeks, Examples 1 to 5 exhibited a similar level of protein synthesis capability to that of the protein synthase solution of Comparative Example 2.

On the other hand, in the case of Comparative Example 1, in which all of the constituent liquids were mixed together prior to frozen storage, the level of protein synthesis capability deteriorated considerably as the storage period lengthened. It is surmised that this is because the enzyme contained within the constituent liquid was in a state of coexistence with first components capable of reacting with the enzyme.

Further, in Examples 1 to 5, reaction was able to be performed quickly, with no loss of the constituent liquids.

The above results confirmed that the enzymatic reaction reagent of the present invention was simple to operate and exhibited an excellent level of protein synthesis capability.

### INDUSTRIAL APPLICABILITY

The present invention can be used favorably in the fields of medical diagnosis or material production.

## Claims

1. An enzymatic reaction reagent prepared by freezing a liquid for enzymatic reaction that is divided into a plurality of constituent liquids,
wherein
the constituent liquid is one of a plurality of separated liquid units,
at least one of the constituent liquids comprises extracts obtained from microbes or cells, which contain the targeted enzyme,
each constituent liquid contains a vehicle,
each of the constituent liquids is frozen individually,
the plurality of constituent liquids are frozen in a state of mutual contact, or the constituent liquids are each formed as a frozen layer in a stacked configuration,
a first component capable of reacting with the enzyme is provided within a constituent liquid that does not comprise the enzyme, and
all of the constituent liquids are encased in a single container.

2. The enzymatic reaction reagent according to claim 1, wherein a second component, which is different from the first component and is capable of reducing activity of the enzyme, is provided within a constituent liquid that does not comprise the enzyme.

3. The enzymatic reaction reagent according to claim 1, wherein the reagent is used for protein synthesis.

4. An enzymatic reaction reagent kit, comprising the enzymatic reaction reagent according to any one of claims 1 to 3.

5. A method for storing a liquid for an enzymatic reaction that has been divided into a plurality of constituent liquids, wherein
the constituent liquid is one of a plurality of separated liquid units,
at least one of the constituent liquids comprises extracts obtained from microbes or cells, which contain the targeted enzyme, each constituent liquid contains a vehicle, each of the constituent liquids is frozen individually in succession, the plurality of constituent liquids are frozen in a state of mutual contact, or the constituent liquids are each formed as a frozen layer in a stacked configuration, a first component capable of reacting with the enzyme is provided within a constituent liquid that does not comprise the enzyme, all of the constituent liquids are encased in a single container, and the container is stored in a frozen state.

6. The enzymatic reaction reagent according to claim 1, wherein the vehicle is a solvent for a component.

7. The enzymatic reaction reagent according to claim 1, wherein the vehicle is water.

8. The method for storing a liquid for an enzymatic reaction according to claim 5, wherein the vehicle is a solvent for a component.

9. The method for storing a liquid for an enzymatic reaction according to claim 5, wherein the vehicle is water.

## Patentansprüche

1. Enzymreaktionsreagens, hergestellt durch Einfrieren einer Flüssigkeit für eine enzymatische Reaktion, die in mehrere flüssige Bestandteile geteilt ist,
wobei
der flüssige Bestandteil eine von mehreren getrennten flüssigen Einheiten ist, zumindest einer der flüssigen Bestandteile Extrakte umfasst, die aus Mikroben oder Zellen erhalten werden, die das Zielenzym enthalten,
jeder flüssige Bestandteil ein Vehikel enthält,
jeder der flüssigen Bestandteile einzeln eingefroren wird,
die mehreren flüssigen Bestandteile in einem Zustand gegenseitigen Kontakts eingefroren werden, oder die flüssigen Bestandteile jeweils als gefrorene Schicht in einer gestapelten Anordnung ausgebildet werden,
eine erste Komponente, die zum Reagieren mit dem Enzym fähig ist, innerhalb eines flüssigen Bestandteils bereitgestellt wird, der das Enzym nicht umfasst, und
alle der flüssigen Bestandteile in einem einzigen Behälter eingeschlossen werden.

2. Enzymreaktionsreagens nach Anspruch 1, wobei eine zweite Komponente, die sich von der ersten Komponente unterscheidet und zum Verringern von Aktivität des Enzyms fähig ist, innerhalb eines flüssigen Bestandteils bereitgestellt wird, der das Enzym nicht umfasst.

3. Enzymreaktionsreagens nach Anspruch 1, wobei das Reagens für Proteinsynthese verwendet wird.

4. Enzymreaktionsreagenskit, das das Enzymreaktionsreagens nach einem der Ansprüche 1 bis 3 umfasst.

5. Verfahren zur Lagerung einer Flüssigkeit für eine enzymatische Reaktion, die in mehrere flüssige Bestandteile geteilt worden ist, wobei
der flüssige Bestandteil eine von mehreren getrennten flüssigen Einheiten ist, zumindest einer der flüssigen Bestandteile Extrakte umfasst, die aus Mikroben oder Zellen erhalten werden, die das Zielenzym enthalten, jeder flüssige Bestandteil ein Vehikel enthält, jeder der flüssigen Bestandteile nacheinander einzeln eingefroren wird, die mehreren flüssigen Bestandteile in einem Zustand gegenseitigen Kontakts eingefroren werden, oder die flüssigen Bestandteile jeweils als gefrorene Schicht in einer gestapelten Anordnung ausgebildet werden, eine erste Komponente, die zum Reagieren mit dem Enzym fähig ist, innerhalb eines flüssigen Bestandteils bereitgestellt wird, der das Enzym nicht umfasst, alle der flüssigen Bestandteile in einem einzigen Behälter eingeschlossen werden, und der Behälter in Gefrierzustand gelagert wird.

6. Enzymreaktionsreagens nach Anspruch 1, wobei das Vehikel ein Lösungsmittel für eine Komponente ist.

7. Enzymreaktionsreagens nach Anspruch 1, wobei das Vehikel Wasser ist.

8. Verfahren zur Lagerung einer Flüssigkeit für eine enzymatische Reaktion nach Anspruch 5, wobei das Vehikel ein Lösungsmittel für eine Komponente ist.

9. Verfahren zur Lagerung einer Flüssigkeit für eine enzymatische Reaktion nach Anspruch 5, wobei das Vehikel Wasser ist.

## Revendications

1. Réactif de réaction enzymatique préparé par la congélation d'un liquide pour une réaction enzymatique qui est divisé en une pluralité de liquides constituants,
dans lequel:
le liquide constituant est une parmi une pluralité d'unités liquides séparées,
au moins un des liquides constituants comprend des extraits obtenus à partir de microbes ou de cellules, qui contiennent l'enzyme ciblée,
chaque liquide constituant contient un véhicule,
chacun des liquides constituants est congelé individuellement,
la pluralité des liquides constituants est congelée dans un état de contact mutuel ou les liquides constituants sont chacun formés comme une couche congelée dans une configuration empilée,
un premier composant capable de réagir avec l'enzyme est fourni dans un liquide constituant qui ne comprend pas l'enzyme, et
tous les liquides constituants sont placés dans un seul récipient.

2. Réactif de réaction enzymatique selon la revendication 1, dans lequel un deuxième composant, qui est différent du premier composant et qui est capable de réduire l'activité de l'enzyme, est fourni dans un liquide constituant qui ne comprend pas l'enzyme.

3. Réactif de réaction enzymatique selon la revendication 1, où le réactif est utilisé pour une synthèse de protéines.

4. Kit de réactif de réaction enzymatique, comprenant le réactif de réaction enzymatique selon l'une quelconque des revendications 1 à 3.

5. Procédé pour le stockage d'un liquide pour une réaction enzymatique qui a été divisé en une pluralité de liquides constituants, dans lequel:
le liquide constituant est une parmi une pluralité d'unités liquides séparées,
au moins un des liquides constituants comprend des extraits obtenus à partir de microbes ou de cellules, qui contiennent l'enzyme ciblée, chaque liquide constituant contient un véhicule, chacun des liquides constituants est congelé individuellement successivement, la pluralité de liquides constituants est congelée dans un état de contact mutuel ou les liquides constituants sont chacun formés comme une couche congelée dans une configuration empilée, un premier composant capable de réagir avec l'enzyme est fourni dans un liquide constituant qui ne comprend pas l'enzyme, tous les liquides constituants sont placés dans un seul récipient et le récipient est stocké dans un état congelé.

6. Réactif de réaction enzymatique selon la revendication 1, dans lequel le véhicule est un solvant pour un composant.

7. Réactif de réaction enzymatique selon la revendication 1, dans lequel le véhicule est de l'eau.

8. Procédé pour le stockage d'un liquide pour une réaction enzymatique selon la revendication 5, dans lequel le véhicule est un solvant pour un composant.

9. Procédé pour le stockage d'un liquide pour une réaction enzymatique selon la revendication 5, dans lequel le véhicule est de l'eau.
